# EUROPEAN PATENT APPLICATION

(11) **EP 4 331 664 A1**
(43) Date of publication of application: **06.03.2024**
(21) Application number: 23192989.4
(22) Date of filing: 23.08.2023
(51) Int. Cl.: A61N 5/10, G06F 3/16, G16H 20/40

(54) **A SYSTEM FOR MONITORING POSITION OF A PATIENT**

(30) Priority: 31.08.2022 EP 22193158
(71) Applicant: Vision RT Limited, London N3 2JU (GB)
(72) Inventor: BARRETT, Adrian Roger William, London, N3 2JU (GB); WAGHORN, Benjamin James, London, N3 2JU (GB)
(74) Representative: Demant

(57) **Abstract**

The present invention inter alia relates to a monitoring system (100) for use with a medical apparatus (210) to monitor the position of a patient (220), the monitoring system (100) comprising:
- at least one visual sensor (110) providing visual data;
- at least one processing unit (120) configured to generate, based on the visual data, one or more views; and
- at least one display (130) for displaying the one or more generated views.

The object of providing a monitoring system that allows a more convenient and hands-free operation is solved in that the monitoring system (100) further comprises at least one acoustic sensor (140) providing a signal, wherein the at least one processing unit (120) is configured to perform an action based on the signal of the at least one acoustic sensor (140).

## Description

The present invention relates to a patient monitoring system for use together with a medical device. In particular, the medical device is of a type that requires precise placement of a patient to ensure correct treatment as well as optimal tomography and/or simulation. In addition, the invention relates to a patient monitoring system for use in radiotherapy treatment, computerized tomography and/or medical simulation, and to a method for monitoring the position of a patient during treatment, imaging, or simulation.

In radiotherapy, a radiation beam emitted by a radiation source is projected onto a predetermined region of a patient's body for a certain amount of time, usually in order to destroy or eliminate tumours existing therein. A radiotherapy treatment is typically carried out in several treatment sessions happening in regular intervals. For each session, a therapist must carefully position the patient with respect to the radiation beam in order to ensure that the selected body region is irradiated with the highest possible accuracy. Otherwise, healthy tissue around the selected body region may be damaged by the radiation.

Radiotherapy apparatuses of prior art are calibrated such that the radiation beam is focused on a so-called treatment iso-centre. The patient usually lies on a couch which can be positioned in all three directions of space by mechanical drives. It is then a task of the therapist to adjust the position of the couch such that the iso-centre of the radiotherapy apparatus coincides with the patient's body region to be treated. The patient's body, or parts thereof, can be fixed to the couch to avoid movements by the patient during the treatment session.

For assisting the therapist in correctly positioning the couch with the patient for a respective radiotherapy treatment, monitoring systems to monitor the patient's position are available. A conventional monitoring system, such as the monitoring system described in patent document WO 2016/181156 A1, typically comprises a visual sensor, a processing unit, and a display. The visual sensor provides visual data and may be, for example, a stereoscopic camera. The camera may be suspended from the ceiling of a treatment room in a fixed location, such as located approximately 1.5 m to 2 m away from the patient or may be mounted on the couch. The patient on the couch is located at a known position, which enables calibration of the camera such that the relative position of the patient relative to the treatment iso-centre may be identified. The processing unit typically generates different views of the patient and/or of the radiotherapy apparatus based on the visual data provided by the visual sensor. The views may be, for example, a three-dimensional view of the patient's body surface or a real-time video view of the patient's body. The different generated views are shown on the display to enable the patient to be correctly positioned by the therapist with the aid of the monitoring system.

During the positioning procedure, the therapist usually must observe the display to assess both the current patient position and the desired patient position, until both of them coincide. Furthermore, the therapist often must switch between different views shown on the display, for example in order to cross-check the patient position in different views. The switching is typically done via input devices like a computer keyboard or mouse of the processing unit, which generally are located away from the radiotherapy apparatus in the treatment room. Thus, the switching between the views is currently a cumbersome procedure, because it requires the therapist to turn away from the patient or even in some instances to leave the patient, go to the keyboard and mouse to select a different view and then return to the patient. The same also applies to the overall operation of the monitoring system, for example starting and/or stopping a real-time monitoring of the patient's position. In total, this negatively affects the therapist/patient interaction, introduces workflow inefficiencies, and prevents the therapist from facing the patient. In addition, the therapist's hands are occupied for the operation of the monitoring systems.

The problems described above occur in a similar manner also with other medical apparatuses as soon as the patient's position plays an important role. When performing a computed tomography (CT) scan, for example, a precise positioning of the patient may be helpful to obtain an appropriate image. In this context, monitoring systems are used, for example, to position the patient before the CT scan, to track respiratory motion by the patient during the CT scan, or to capture the full surface of the patient at the time of the CT scan, since CT usually only images a part of the patient's body. For the operation of such monitoring systems, for example during the CT scan, or during a setup procedure of the patient that is performed before the CT scan, it is at present a widespread practice to use input devices such as a computer keyboard or mouse. However, similarly to what has been explained above in the context of radiotherapy, this negatively affects the therapist/patient interaction, introduces workflow inefficiencies, prevents the therapist from facing the patient, and occupies the therapist's hands.

Against this background, an object of the present invention is to provide a monitoring system for use with a medical device configured to provide radiotherapy treatment, computed tomography, or simulation that enables a more convenient and hands-free operation. In addition, an object of the invention is to specify an improved monitoring system comprising voice control. A first aspect of the present invention relates to a monitoring system for use with a medical device, such as configured to provide radiotherapy treatment, computed tomography, or simulation, the monitoring system comprising:
- at least one visual sensor providing visual data;
- at least one processing unit configured to generate, based on the visual data, one or more views; and
- at least one display for displaying the one or more generated views; and
- at least one acoustic sensor providing a signal; and
wherein the at least one processing unit is configured to analysing the signal of the at least one acoustic sensor and based thereon to selecting an action corresponding to an operative response of the monitoring system.

The term simulation is in this context used for planning radiotherapy so that the target is precisely located and marked. This may involve taking a CT scan of the patient and using this in a Treatment Planning System (TPS). It may also involve placing marks on the patient's body (for subsequent alignment) and making devices too help with positioning (e.g., masks, vacuum bags) or to change dose characteristics (e.g., bolus materials).

According to the first aspect, the above object is solved for such a monitoring system in that the monitoring system further comprises at least one acoustic sensor providing a signal, wherein the at least one processing unit is configured to perform an action based on the signal of the at least one acoustic sensor. Further, according to the first aspect, the above object is solved for such a monitoring system in that the therapist/patient interaction is improved, workflow inefficiencies are reduced, and the therapist's hands are no longer occupied when operating the monitoring system,

The monitoring system according to the first aspect comprises at least one visual sensor providing visual data. In particular, the at least one visual sensor may be at least one surface imaging camera. In general, however, any visual sensor providing suitable visual data, including a thermographic camera or the like, is conceivable. Further, the visual sensor may comprise stereoscopic camera, LIDAR sensor, time-of-flight sensor, laser scanner, and/or structured light scanner. By the at least one visual sensor, it is achieved that visual data for the generation of the one or more views is provided. By at least one stereoscopic camera, it is achieved that using the principles of stereoscopy, visual data enabling a three-dimensional representation of the patient is provided.

The monitoring system according to the first aspect further comprises at least one processing unit configured to generate, based on the visual data, one or more views. The at least one processing unit may be an internal processing unit of the monitoring system, or it may be an external processing unit, for example a general-purpose personal computer that is connected by wires or wirelessly to the at least one visual sensor and that runs a respective computer program, or a combination of both. A view in this context is understood to mean any suitable visual representation of an object, such as a patient or a phantom, or parts thereof, and/or of an associated apparatus, or parts thereof, that may aid a therapist in correctly positioning a patient for a medical procedure, such as for example a radiotherapy treatment, a CT scan, and or medical simulation. A view may be a realistic representation, similar to a photograph or video, or it may be merely a schematic representation. Different views may also refer to representations of the same object from different perspectives. The generation of the one or more views based on the visual data is understood to mean that the visual data is used in any suitable way to generate the one or more views such as different perspectives of the same object. For example, the visual data may be processed, highlights may be added, projections, scales, markers, colour codes may be introduced, etc. By the at least one processing unit configured to generate, based on the visual data, one or more views, it is achieved that one or more views for aiding the therapist in correctly positioning the patient can be provided to the therapist.

Further, a phantom may be in the form of a calibration cube containing irradiation targets, such as one or more small ceramic or metallic balls or other ceramic or metal targets such as targets made of steel, titanium or tungsten or the like. The phantom may be positioned with the phantom's center at an estimated location for the iso-center of a radiotherapy treatment apparatus with the choice of material being dependent upon the nature of the radiation being applied by the treatment apparatus (e.g. ceramic targets may be imaged utilizing MV or kV radiation whereas metal targets show up better when radiated with MV radiation but worse when irradiated with kV radiation). The calibration phantom is then irradiated using the radiotherapy treatment apparatus. The relative location of the center of the calibration phantom and the iso-center of the radiotherapy treatment is then determined by analyzing radiographic images of the irradiation of the calibration phantom containing the irradiation targets.

The monitoring system according to the first aspect further comprises at least one display for displaying the one or more generated views. For example, the at least one display may be connected to a graphics output of the at least one processing unit. By the at least one display, it is achieved that the generated views for aiding the therapist in correctly positioning the patient can be optically perceived by the therapist.

It has been recognised in the context of the first aspect that by using at least one acoustic sensor providing a signal, and by configuring the at least one processing unit such that the at least one processing unit performs an action based on the signal of the at least one acoustic sensor, a convenient and hands-free operation of the monitoring system can be provided to the therapist. In this context, performing an action based on the signal of the at least one acoustic sensor is understood to mean that the signal of the at least one acoustic sensor is used in any suitable way to identify the action to be performed. For example, the at least one acoustic sensor may be in particular at least one microphone. The therapist may, for example, produce certain sounds, such as using his voice, and these sounds may be obtained or received by the at least one acoustic sensor as input. The sounds may, at least partially, be included in the signal output by the at least one acoustic sensor and may be analysed, processed or identified by the at least one processing unit in order to perform an action. Thus, it is no longer required that the therapist uses a computer keyboard or mouse for operating the monitoring system, and accordingly, the therapist/patient interaction is improved, workflow inefficiencies are reduced, and the therapist's hands are no longer occupied when operating the monitoring system. In addition, the risk of infections is decreased since physical contact with the computer keyboard or mouse is reduced.

The signal of the at least one acoustic sensor may in this context be construed as an analogue or digital (electric) signal representing the acoustic sound captured by the at least one acoustic sensor. In other words, the signal of the at least one acoustic sensor may be a signal representing acoustic information captured by the at least one acoustic sensor. The signal may also undergo certain processing steps in the at least one processing unit or in other units, such that a processed signal is generated, which may be used for identifying the action to be performed.

A second aspect of the present invention relates to use of the monitoring system according to the first aspect of the invention, wherein the monitoring system is used with a medical device configured to provide radiotherapy treatment, computed tomography, or simulation.

According to the second aspect, the above object is solved for such a radiotherapy system in that the monitoring system according to the first aspect. Thereby, the therapist/patient interaction is improved, workflow inefficiencies are reduced, and the therapist's hands are no longer occupied when operating the monitoring system, as has already been explained in the context of the first aspect.

A third aspect of the present invention relates to a method for monitoring the position of a patient with a monitoring system of a medical apparatus, the method comprising:
- providing visual data by at least one visual sensor;
- generating, based on the visual data, by at least one processing unit, one or more views; and
- displaying the one or more generated views on a display.

According to the third aspect, the above object is solved for such a method in that the method further comprises:
- providing a signal by at least one acoustic sensor (140); and
- analysing the signal (140) and based thereon selecting an action corresponding to an operative response of the monitoring system by the at least one processing unit (120).

Thereby, the therapist/patient interaction is improved, workflow inefficiencies are reduced, and the therapist's hands are no longer occupied when operating the monitoring system, as has already been explained in the context of the first aspect.

In an embodiment of the different aspects, the at least one processing unit is configured to apply a voice recognition algorithm on the signal of the at least one acoustic sensor to select an action corresponding to an operative response of the monitoring system based thereon. Thereby, convenience for the therapist is further improved because the therapist may use ordinary conversational speech to operate the monitoring system.

In an embodiment of the different aspects, the voice recognition algorithm is configured to recognise predefined combinations of at least one keyword and at least one command phrase within the signal of the at least one acoustic sensor. Thereby, the reliability of the operation of the monitoring system can be enhanced, and convenience can be improved even further.

In an embodiment of the different aspects, two or more different views are generated based on the visual data. For the monitoring system according to the second aspect and as used in the first aspect, this is understood to mean that the at least one processing unit is configured to generate, based on the visual data, two or more different views. For the method according to the third aspect, this is understood to mean that the method comprises generating, based on the visual data, by the at least one processing unit, two or more different views. By generating two or more different views based on the visual data, additional data can be provided to the therapist, such that in particular the positioning of the patient can be facilitated.

In an embodiment of the different aspects where two or more different views are generated based on the visual data, the action that is performed based on the signal of the at least one acoustic sensor comprises a selection, based on the signal of the at least one acoustic sensor of a specific generated view to be displayed on the at least one display. In particular, the selection provides a specific generated view to be displayed on the at least one display, which establishes a convenient and hands-free operation of the monitoring system can be provided to the therapist. This is because during typical operation of a monitoring system, a regularly occurring action which requires the therapist to leave the patient is the switching between different views. Further, the selection provides a specific generated view to be displayed on the at least one display, which ensures that the therapist no longer is required to use a computer keyboard or mouse to switch between the different views. Accordingly, the therapist/patient interaction is further improved, workflow inefficiencies are further reduced, and the therapist's hands are no longer occupied, when switching views during operation of the monitoring system.

In an embodiment of the different aspects, the views generated by the at least one processing unit based on the visual data comprise a three-dimensional partial or full surface view of the patient. A three-dimensional surface view of the patient is understood to mean a representation of the three-dimensional surface topography of the patient or parts thereof. The three-dimensional surface view may be realised, for example, by a mesh representation of the surface topography of the patient. The three-dimensional surface view may be realised, as another example, by a representation of a rendered surface of the patient generated from a three-dimensional model of the patient. The three-dimensional surface view may include photographs of the patient using texture mapping. A three-dimensional surface view of the patient provides the advantage that the different body parts of the patient and their position and alignment in space can easily be identified, which helps in positioning the patient.

In an embodiment of the different aspects, the views generated by the at least one processing unit based on the visual data comprise a partial or full surface deformation view of the patient. A surface deformation view of the patient is understood to mean a representation of the patient or parts thereof that shows the degree of concordance of a surface of the patient with a predetermined reference surface. For example, surface regions where the surface of the patient coincides with the reference surface within a predefined tolerance range may be displayed in a first colour, whereas surface regions where the difference between the surface of the patient and the reference surface exceeds the tolerance range are displayed in a second and/or third colour. For example, green colour may be used to indicate coincidence between the surface of the patient and the reference surface within the tolerance range, red colour may be used to indicate that the surface of the patient is above the reference surface to a degree that exceeds the tolerance range, and blue colour may be used to indicate that the surface of the patient is below the reference surface to a degree that exceeds the tolerance range. The predetermined reference surface may represent, for example, a desired patient position. In this case, a surface deformation view of the patient provides the advantage that it additionally aids in correctly positioning the patient. Alternatively, in the case of radiotherapy, the predetermined reference surface may represent, for example, a previous surface topography of the patient that has been determined, for example, based on measurements performed during a preceding treatment session. In this case, a surface deformation view of the patient provides the advantage that it allows to assess whether and to what degree the surface topography of the patient, particularly the body part to be treated with radiotherapy, has changed, for example resulting from tumour shrinkage, and may in addition indicate the need for a replanning of the treatment.

In an embodiment of the different aspects, the views generated by the at least one processing unit based on the visual data comprise a real-time video view of the patient's body. The real-time video view may show the complete body of the patient, or only parts thereof. A real-time video view of the patient provides the advantage that a realistic view of the patient can be provided to the therapist, which aids in correctly positioning the patient. The real-time video view may additionally show patient outlines. This provides the advantage that positioning of the patient can be facilitated further, since the outlines provide visual orientation to the therapist regarding the position of the patient.

In an embodiment of the different aspects, the views generated by the at least one processing unit based on the visual data comprise an outline view of the patient. An outline view of the patient is understood to mean a representation of the patient's body or parts thereof that only shows patient outlines. This also provides the advantage that positioning of the patient can be facilitated.

In an embodiment of the different aspects, the views generated by the at least one processing unit based on the visual data comprise an accessory's view. An accessory's view is understood to mean a view of at least one accessory required for a medical procedure, such as for example a radiotherapy treatment or a CT scan, together with an indication where the at least one accessory is to be positioned. In particular in radiotherapy, potential accessories may comprise, for example, patient positioning devices for supporting the patient and minimising movements, such as breast boards, wing boards, vac-lok bags, thermoplastic masks or knee sponges, or beam attenuating devices for modifying the characteristics of the radiation delivery to the target, such as bolus elements. An accessory's view provides the advantage that the potential accessories required for the treatment can also be correctly positioned by the therapist in a convenient way.

In an embodiment of the different aspects, the action that is performed based on the signal of the at least one acoustic sensor comprises starting and/or stopping, based on the signal of the at least one acoustic sensor, a real-time monitoring of the position of the patient. Thereby, a convenient and hands-free operation of the monitoring system can likewise be provided to the therapist since the therapist does not have to leave the patient for turning on and/or off real-time monitoring of the patient's position. The controlling of starting and/or stopping of the real-time monitoring based on the signal of the at least one acoustic sensor, therefore alleviates that the therapist uses a computer keyboard or mouse to turn on and/or off the real-time monitoring. Accordingly, the therapist/patient interaction is further improved, workflow inefficiencies are further reduced, and the therapist's hands are no longer occupied, when turning on and/or off real-time monitoring.

In an embodiment of the different aspects, the monitoring system further comprises at least one input device for controlling the at least one processing unit. The at least one input device may be, for example, at least one computer keyboard and/or at least one computer mouse. In particular, the at least one input device is connected to the at least one processing unit. By the at least one input device, a backup system for switching view to position the patient can be provided. For example, in the case of a malfunction or defect of the at least one acoustic sensor, the therapist can use the at least one input device for selecting which view is to be displayed on the display.

In an embodiment of the different aspects, the medical apparatus is a radiotherapy apparatus. For the monitoring system according to the first aspect, this is understood to mean that the monitoring system is configured for use with a radiotherapy apparatus. For the method according to the third aspect, this is understood to mean that the method is performed with a monitoring system of a radiotherapy apparatus. With the medical apparatus being a radiotherapy apparatus, the advantages of the invention can be achieved in radiotherapy, where a precise positioning of the patient is of special importance. As already explained above, the advantages of the invention include an improved therapist/patient interaction, reduced workflow inefficiencies, and the implementation of a hands-free operation of the monitoring system.

In an embodiment of the different aspects, the medical apparatus is a computed tomography apparatus, also known as CT scanner. For the monitoring system according to the first aspect, this is understood to mean that the monitoring system is configured for use with a computed tomography apparatus. For the method according to the third aspect, this is understood to mean that the method is performed with a monitoring system of a computed tomography apparatus. With the medical apparatus being a computed tomography apparatus, the advantages of the invention can be achieved in computed tomography, where a precise positioning of the patient likewise plays an important role. As already explained above, the advantages of the invention include an improved therapist/patient interaction, reduced workflow inefficiencies, and the implementation of a hands-free operation of the monitoring system.

The disclosure of means for performing a method step is to be understood to also disclose the respective method step. Likewise, the disclosure of a method step is to be understood to also disclose the respective means for performing the method step.

Further configurations and advantages of the invention will be explained in the following detailed description of some exemplary embodiments of the present invention in conjunction with the drawings. In the drawing,
- Fig. 1: shows a schematic view of a monitoring system according to the first aspect of the present invention; and
- Fig. 2: shows a schematic view of a system according to the second aspect of the present invention.

In the following, exemplary embodiments of the invention are described in the context of a monitoring system for a radiotherapy apparatus, as an example. However, this is not intended to be understood such that the invention is limited only to radiotherapy. Instead, a person skilled in the art will be able to readily implement the invention also into monitoring systems for other types of medical apparatuses requiring a great degree of precision of patient positioning in the medical apparatuses, such as a monitoring system for a computed tomography apparatus.

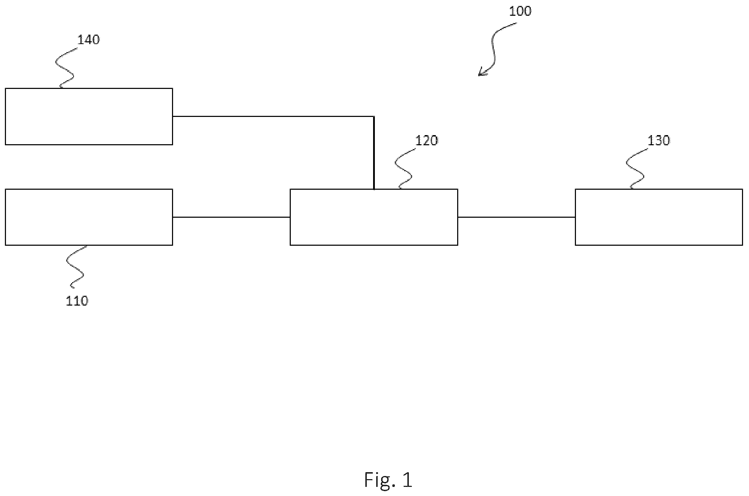

Referring first to Fig. 1, a monitoring system 100 according to the first aspect is shown in a schematic view. The monitoring system 100 comprises a visual sensor 110, here a stereoscopic camera 110, as well as a processing unit 120 and a display 130. The visual sensor 110 records visual data and provides the visual data to the processing unit 120. Based on the received visual data, the processing unit 120 creates one or more views of a patient who is to be treated with radiotherapy and of a radiotherapy apparatus. The views are sent to the display 130 and displayed there. Like this, a therapist conducting the radiotherapy treatment is aided in correctly positioning the patient.

The monitoring system 100 further comprises an acoustic sensor 140, here a microphone 140, which records a signal of the acoustic sensor, for example from the treatment room, and provides the signal of the acoustic sensor to the processing unit 120. Based on the received signal of the acoustic sensor, the processing unit 120 activates and/or deactivates a real-time monitoring of the patient and decides which of the different views is displayed on the display 130. In this example, the signal of the acoustic sensor is analysed by the processing unit 120 using a voice recognition algorithm. This enables the therapist in a convenient and hands-free way to switch between different views to be displayed.

Further, the processing unit 120 comprises a memory for storing keywords associated with a variety of actions relating to operational control of the monitoring system. The acoustic sensor records a voice instruction of a therapist and generates a signal, which is forwarded to the processing unit 120. The processing unit 120 analyses the signal, for example by means of voice recognition, and compares the analysis with a bank of keywords in the memory. In case the processing unit 120 identifies a match the, the processing unit 120 activates a task associated with the identified keyword that causes an operational control of the monitoring system 100.

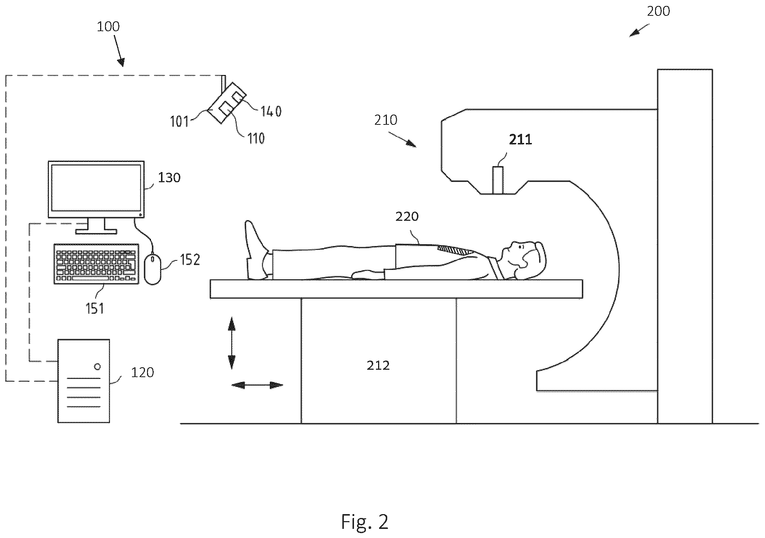

In Fig. 2, the monitoring system 100 is shown as used together with a radiotherapy apparatus 210according to the second aspect. The radiotherapy apparatus 210 comprises a radiation source 211 which is directed onto a patient 220 for a radiotherapy treatment session. The patient 220 lies on a couch 212 which can be moved in all three directions of space, two of which being indicated by the arrows in Fig. 2. Additionally, the couch 212 may be rotated in all three directions of space (not shown).

Before starting the radiotherapy treatment, a therapist (not shown) must take care of the correct positioning of the patient 220. For this purpose, the monitoring system 100 is used, as described in the following. The monitoring system 100 comprises a sensor unit 101 which is suspended from the ceiling of the treatment room. In other embodiments of the invention, the sensor unit may be mounted on the couch for the patient. The sensor unit 101 comprises, in the example embodiment described here, a total of three stereoscopic cameras 110, of which only one is shown in Fig. 2. The three stereoscopic cameras 110 are arranged such that a central camera is located in the symmetry plane of the patient, whereas the remaining two side cameras are shifted to the left and right side of the patient with respect to the central camera.

The sensor unit 101 is connected to a personal computer 120 which comprises a processor (not shown) and a memory (not shown) and acts as a processing unit 120 for the monitoring system 100 by running a respective computer program. Like this, the image data provided by the stereoscopic cameras 110 is processed into different views for aiding the therapist in positioning the patient 220. The views generated in this way are displayed on the display 130 to be viewed by the therapist.

In this example, the following views are created from the image data provided by the three stereoscopic cameras 110:
- a three-dimensional surface view of the patient;
- a surface deformation view of the patient;
- a real-time video view of the patient, optionally including patient outlines;
- an outline view of the patient;
- a view of accessories required for the treatment together with an indication where the accessories are to be positioned.

With respect to the real-time video view, it can be selected whether the video image from the left, central or right stereoscopic camera 110 is to be displayed. The same applies to the outline view and to the view of accessories. Thereby, in this example embodiment, a total of several different views can be provided to the therapist to aid in correctly positioning the patient 220 and, if required, the accessories.

For activating and/or deactivating real-time monitoring of the patient, and for switching between the views, the sensor unit 101 also comprises a microphone 140 which is likewise connected to the personal computer 120 either via a wired or wireless connection. In an alternative embodiment the microphone 140 may be attached to the shirt of the therapist and communicate with the personal computer 120, for example by using a Bluetooth connection. The signal of the acoustic sensor provided by the microphone 140 is analysed in the computer 120 using a voice recognition algorithm. Depending on the result of the voice recognition, the computer 120 may perform an action, such as activate and/or deactivate real-time monitoring or select a specific view to be displayed on the display 130. In this example, the voice recognition algorithm is set up to recognise predefined combinations of a keyword and a command phrase within the signal of the acoustic sensor. Here, the word "vision" has been predefined as keyword and the phrases shown in table 1 have been defined as command phrases.

**Table 1**

| **Command phrase** | **Assigned view or action** |
|---|---|
| "start" | Start live monitoring |
| "stop" | Stop live monitoring |
| "surface" | Three-dimensional surface view |
| "deformation" | Surface deformation view |
| "video" | Real-time video view |
| "outlines only" | Outline view |
| "accessory" | Accessory's view |
| "pod 1" | Select left camera in real-time video view, outline view or accessory's view |
| "pod 2" | Select right camera in real-time video view, outline view or accessory's view |
| "pod 3" | Select central camera in real-time video view, outline view or accessory's view |

For example, when the voice recognition algorithm recognizes that the therapist expresses the phrase "vision start" with his voice, the computer 120 will start the real-time monitoring of the position of the patient. Similarly, when the phrase "vision surface" is recognised, the computer 120 will display the three-dimensional surface view on the display 130. Likewise, when the phrase "vision video" is recognised, the real-time video view, in this example of the camera that has been selected most recently, or if no camera has been selected yet, of the central camera, will be displayed, and so forth. The use of a keyword, here "vision", provides the advantage that an assigned action is only performed if the keyword has been expressed, too. This avoids unintended performing of actions, for example if the command phrases are expressed within a normal conversation. Accordingly, convenience for the therapist is improved.

The definition of further command phrases, other than those included in table 1, to select other views or to perform other actions is of course conceivable. For example, the command phrase "outlines on" may be assigned to activating the display of patient outlines in the real-time video view, the command phrase "outlines off" may be assigned to deactivating the display of patient outlines in the real-time video view, and the command phrase "screenshot" may be assigned to take a screenshot of the current view. Further, examples of the command phrases may be "capture surface" for establishing a surface of a patient or phantom, "change reference" for ...., "change region of interest" for zooming to a specific location on the patient. However, these are only selected examples and in principle, any action performed by the monitoring system can be triggered based on the voice recognition of a respectively assigned command phrase. Furthermore, whereas the keyword and command phrases in the example embodiment described here are in English language, it is readily conceivable to use, additionally or alternatively, keywords and command phrases in any other language.

For correctly positioning the patient 220, the therapist can then proceed as follows: After laying down the patient 220 on the couch 212, the therapist can activate real-time monitoring and select a first view by expressing a keyword and the respective command phrases, as explained above. Using the first view, the therapist can then start adjusting the position and alignment of the patient 220 in all three directions of space using the movable couch 212. During the positioning, the therapist can switch to a different view by expressing again the keyword and the respective command phrase. Like this, the therapist can use his voice for switching between the different views to aid in positioning the patient 220 until the current patient position and the desired patient position coincide. In addition, if any accessories are required for the treatment, the therapist can switch to the accessory view using his voice and correctly position the accessories.

Because the activation and/or deactivation of the real-time monitoring, as well as the selection of the specific generated view to be displayed on the display 130, is performed by the computer 120 based on the signal of the acoustic sensor, here based on the result of the voice recognition, a convenient and hands-free operation of the monitoring system is enabled. This applies to the switching between the different views by the therapist. More specifically, it is no longer required that the therapist uses a computer keyboard or mouse to operate the monitoring system, and accordingly, the therapist/patient interaction is improved, workflow inefficiencies are reduced, and the therapist's hands are no longer occupied.

Still, the monitoring system 100 in Fig. 2 also comprises a computer keyboard 151 and a mouse 152 as input devices for controlling the computer 120. The keyboard 151 and mouse 152 serve as a backup system here. In the case of a malfunction or defect of the microphone 140, the therapist may use the keyboard 151 and mouse 152 for operating the monitoring system, and for selecting which view is to be displayed on the display 130.

## Claims

1. A monitoring system (100) for use with a medical apparatus (210) to monitor the position of a patient (220), the monitoring system (100) comprising:
- at least one visual sensor (110) providing visual data;
- at least one processing unit (120) configured to generate, based on the visual data, one or more views; and
- at least one display (130) for displaying the one or more generated views;
**characterised in that**
the monitoring system (100) further comprises at least one acoustic sensor (140) providing a signal, wherein the at least one processing unit (120) is configured to perform an action based on the signal of the at least one acoustic sensor (140).

2. The monitoring system according to claim 1,
**characterised in that**
the at least one processing unit (120) is configured to apply a voice recognition algorithm on the signal of the at least one acoustic sensor in order to perform an action based on the signal of the at least one acoustic sensor.

3. The monitoring system according to claim 2,
**characterised in that**
the voice recognition algorithm is configured to recognise predefined combinations of at least one keyword and/or at least one command phrase within the signal of the at least one acoustic sensor.

4. The monitoring system according to one of claims 1 to 3,
**characterised in that**
the at least one processing unit (120) is configured to generate, based on the visual data, two or more different views, wherein the action that is performed based on the signal of the at least one acoustic sensor (140) comprises a selection, based on the signal of the at least one acoustic sensor (140), of a specific generated view to be displayed on the at least one display (130).

5. The monitoring system according to one of claims 1 to 4,
**characterised in that**
the views generated by the at least one processing unit (120) based on the visual data comprise a three-dimensional surface view of the patient's body.

6. The monitoring system according to one of claims 1 to 5,
**characterised in that**
the views generated by the at least one processing unit (120) based on the visual data comprise a surface deformation view of the patient's body.

7. The monitoring system according to one of claims 1 to 6,
**characterised in that**
the views generated by the at least one processing unit (120) based on the visual data comprise a real-time video view of the patient's body.

8. The monitoring system according to claim 7,
**characterised in that**
the real-time video view additionally shows patient outlines.

9. The monitoring system according to one of claims 1 to 8,
**characterised in that**
the views generated by the at least one processing unit (120) based on the visual data comprise an outline view of the patient's body.

10. The monitoring system according to one of claims 1 to 9,
**characterised in that**
the views generated by the at least one processing unit (120) based on the visual data comprise a view of at least one accessory required for the treatment together with an indication where the at least one accessory is to be positioned.

11. The monitoring system according to one of claims 1 to 10,
**characterised in that**
the action that is performed based on the signal of the at least one acoustic sensor (140) comprises starting and/or stopping, based on the signal of the at least one acoustic sensor (140), a real-time monitoring of the position of the patient (220).

12. The monitoring system according to one of claims 1 to 11,
**characterised in that**
the monitoring system (100) further comprises at least one input device (151, 152) for controlling the at least one processing unit.

13. The monitoring system according to one of claims 1 to 12,
**characterised in that**
the acoustic sensor (140) is configured to be releasably attached to clothing of an operator of the monitoring system and to be wirelessly connected to the at least one processing unit.

14. Use of the monitoring system according to one of claims 1 to 12, with a radiotherapy apparatus (210) and/or with a computed tomography apparatus.

15. A method for monitoring the position of a patient (220) with a monitoring system (100) of a medical apparatus (210), the method comprising:
- providing visual data by at least one visual sensor (110);
- generating, based on the visual data, by at least one processing unit (120), one or more views; and
- displaying the one or more generated views on a display (130);
**characterised in that**
the method further comprises:
- providing a signal by at least one acoustic sensor (140); and
- based on the signal of the at least one acoustic sensor (140), performing an action by the at least one processing unit (120).

16. The method according to claim 14,
**characterised in that**
the method comprises generating, based on the visual data, by the at least one processing unit (120), two or more different views, wherein the action that is performed based on the signal of the at least one acoustic sensor (140) comprises a selection, based on the signal of the at least one acoustic sensor (140), of a specific generated view to be displayed on the at least one display (130).
